Europäisches Patentamt

European Patent Office

Office européen des brevets

(11) Veröffentlichungsnummer: **0 327 837**
**A2**

# (12) EUROPÄISCHE PATENTANMELDUNG

(21) Anmeldenummer: 89100692.6

(22) Anmeldetag: 17.01.89

(51) Int. Cl.⁴: **G01N 33/04**

(30) Priorität: 08.02.88 DE 3803714

(43) Veröffentlichungstag der Anmeldung:
16.08.89 Patentblatt 89/33

(84) Benannte Vertragsstaaten:
AT CH DE FR GB IT LI NL SE

(71) Anmelder: **BERGPRACHT-MILCHWERK HALDER & CO.**

**D-7992 Tettnang 1 - Siggenweiler(DE)**

(72) Erfinder: **Kurzweil, Richard**
**Siggenweiler 18-2**
**D-7992 Tettnang 1(DE)**
Erfinder: **Halder, Manfred**
**Siggenweiler 37**
**D-7992 Tettnang 1(DE)**

(74) Vertreter: **Engelhardt, Guido, Dipl.-Ing.**
**Patentanwalt Montafonstrasse 35 Postfach 1350**
**D-7990 Friedrichshafen 1(DE)**

(54) Verfahren und Vorrichtung zur Bestimmung der Säuerungsaktivität in Kesselmilch.

(57) Bei einem Verfahren zur Bestimmung der Säuerungsaktivität in Kesselmilch wird einer der zu verarbeitenden Kesselmilch (3) entnommenen Probe ein Redoxindikatorfarbstoff beigegeben. Danach wird die Farbe des Gemisches mittels einer ersten Farbmessung unmittelbar nach der Zugabe des Redoxindikatorfarbstoffes bestimmt und nach einer oder mehreren wählbaren Zeitspannen wird zur Feststellung der Stoffwechseländerungen der Milchsäurebakterien die Farbänderungen der Probe gegenüber der ersten Farbmessungen und/oder vorhergehender Farbmessungen ermittelt.

Auf diese Weise ist es möglich, zu Beginn eines Käsungsprozesses auf das zu erwartende Säuerungsverhalten der zu verarbeitenden Milch zu schließen und entsprechende Maßnahmen zur Steuerung der Säuerungsaktivität rechtzeitig einzuleiten, so daß eine optimale Ausbeute und eine gleichbleibende Qualität des herzustellenden Käses zu erzielen ist.

## Verfahren und Vorrichtung zur Bestimmung der Säuerungsaktivität in Kesselmilch

Die Erfindung bezieht sich auf ein Verfahren sowie eine Vorrichtung zur Bestimmung der Säuerungsaktivität in Kesselmilch, insbesondere in zur Weichkäseherstellung verwendbarer Kesselmilch.

Bei der Herstellung von Käse stellt der Verlauf der Säuerung einen wichtigen Parameter dar, da durch die Säuerung der Geruch, der Geschmack, die Konsistens sowie die Trockenmasse des Käses und damit die Qualität und die Ausbeute beeinflußt werden. Der Säuerungsverlauf, der durch Zugabe von Säurewecker beeinflußt werden kann, ist während des Käsungsprozesses daher von erheblicher Bedeutung.

Um die Säuerungsaktivität der zu verarbeitenden Kesselmilch zu ermitteln, wird nach den bisher in Käsereien angewandten Verfahren die zeitliche pH- und/oder Säuregradänderung in wählbaren Zeitabständen bestimmt. Die pH- und/ oder Säuregradänderungen nach kurzen Zeitintervallen lassen aber über die Säuerungsaktivität nur eine sehr ungenaue Aussage zu, trotz des erheblichen Meßaufwandes.

Die pH- und/oder Säuregradänderungen nach einem längeren Zeitabstand ermöglichen zwar einen guten Aufschluß über die Säuerungsaktivitäten zum Zeitpunkt der ersten Messung, die Ergebnisse dieser später vorzunehmenden Messungen liegen aber zu einem derart späten Zeitpunkt vor, daß eine Beeinflussung durch Änderung der Säurewekkermenge bzw. der Reifungsdauer nicht mehr möglich ist. Erhebliche Ausbeuteverluste und Qualitätsminderungen sind die Folge.

Aufgabe der Erfindung ist es daher, ein Verfahren sowie eine Vorrichtung zu schaffen, durch die es auf sehr einfache Weise und in kurzer Zeit ermöglicht wird, auf das zu erwartende Säuerungsverhalten der zu verarbeitenden Milch zu schließen, um deren Säuerungsaktivität günstig beeinflussen zu können. Der dazu erforderliche Arbeits und Meßaufwand soll gering gehalten werden, auch soll die Durchführung einer Messung ohne Schwierigkeiten und ohne besondere Fachkenntnisse zu bewerkstelligen sein, vor allem aber soll die Messung zu Beginn eines Käsungsprozesses vorgenommen werden können, damit entsprechende Maßnahmen zur Steuerung der Säuerungsaktivität rechtzeitig, gegebenenfalls sofort, einzuleiten sind und somit eine optimale Ausbeute sowie eine gleichbleibende Qualität des herzustellenden Käses zu erzielen ist.

Gemäß der Erfindung ist das Verfahren, mit dem dies zu erreichen ist, dadurch gekennzeichnet, daß einer der zu verarbeitenden Kesselmilch entnommenen Probe ein Redoxindikator-Farbstoff, beispielsweise Resazurin oder Methylenblau, beigegeben wird, daß die Farbe des Gemisches mittels einer ersten Farbmessung unmittelbar nach der Zugabe des Redoxindikator-Farbstoffes bestimmt wird und daß nach einer oder mehreren wählbaren Zeitspannen zur Feststellung der Stoffwechseländerungen der Milchsäurebakterien die Farbänderungen der Probe gegenüber der ersten Farbmessung und/oder vorhergehender Farbmessungen ermittelt wird.

Zweckmäßig ist es hierbei, der Kesselmilch unmittelbar vor dem Einlaben eine Probe zu entnehmen.

Der Redoxindikator-Farbstoff sollte in Form einer Lösung im Verhältnis von ca. 1:10, beispielsweise mittels einer Pipette, der Probe zugegeben, in diese homogen eingemischt und in eine vorgewärmte Küvette eingefüllt werden, wobei der Redoxindikator-Farbstoff im Verhältnis von ca. 1:1000 in vorzugsweise auf 60° erwärmten destillierten Wasser gelöst sein sollte.

Angezeigt ist es des weiteren, die Farbmessungen in gleichen zeitlichen Abständen von vorzugsweise fünf Minuten vorzunehmen, die Küvette zwischen den Farbmessungen, beispielsweise in einem Wärmeschrank, bei einer Temperatur von 30 bis 50° zu halten und die Farbmessungen mittels eines Farbmeßgerätes durchzuführen.

Die Vorrichtung zur Anwendung dieses Verfahrens ist gekennzeichnet durch eine mit einem Rührwerk versehene Küvette, der über eine Zuführungsleitung aus einem Fermeter oder einer Kesselmilchleitung die zu untersuchende Milch vor dem Einlaben und aus einem Vorratsbehälter ein Redoxindikator-Farbstoff gesteuert zuführbar sind, und einem Farbmeßgerät zur Bestimmung der Farbänderungen der in der Küvette hergestellten Mischung.

Vorteilhaft ist es hierbei, die Küvette zur Vornahme von Kalibrierungen verfahrbar, vorzugsweise mittels eines Schlittens auf dem Farbmeßgerät verfahrbar, anzuordnen und in der Zuführungsleitung des RedoxindikatorFarbstoffes eine Dosiereinrichtung vorzusehen.

Das Farbmeßgerät kann an einen Rechner zur Auswertung der Farbmessungen angeschlossen, auch kann mittels des Rechners die Zugabemenge der zu untersuchenden Milch und des Redoxindikator-Farbstoffes in die Küvette gesteuert werden.

Wird die Säuerungsaktivität in Kesselmilch gemäß der Erfindung bestimmt, so ist es möglich, bereits zu Beginn oder nur wenige Minuten nach Beginn eines Käsungsprozesses eine Aussage zu erhalten, aufgrund der mit hoher Sicherheit auf das zu erwartende Säuerungsverhalten der Milch während des Verarbeitungsprozesses geschlossen werden kann. Die Farbänderung der Redoxindikatorfarben in Milch steht nämlich in unmittelbarer Beziehung zur Aktivität der insbesondere für die Käseherstellung notwendigen Milchsäuerebakterien. Durch Zugabe von Säureweckern, Änderung der Säurewecker-Dosierung und/oder der Reifezeit der Milch vor dem Einlaben kann somit zuverlässig, und zwar zu einem frühen Zeitpunkt die Säuerungsaktivität der Milch beeinflußt und damit gesteuert werden.

Durch die Bestimmung der aktuellen Säuerungsaktivität von Kesselmilch vor dem Einlaben und den daraus abzuleitenden Maßnahmen ist es des weiteren möglich, die Produktivität einer Käserei in einem erheblichen Maße zu steigern und bei optimaler Ausbeute Käse gleichbleibender Qualität herzustellen, und zwar trotz der unumgänglichen Schwankungen durch natürliche Ursachen, wie unterschiedliche Milchzusammensetzungen, der verwendeten Bakterienkulturen und anderer Einflüsse. Der Arbeits- und Materialaufwand, mit dem dies zu bewerkstelligen ist, ist äußerst gering, auch sind die Messungen auf einfache Weise und ohne daß Vorkenntnisse erforderlich sind, vorzunehmen und ergeben, insbesondere wenn das Verfahren z. B. mittels eines Rechners automatisiert wird, stets exakte Werte. Die Käseherstellung, vor allem die Herstellung von Weichkäse, kann demnach in einer vorteilhaften Weise verbessert werden.

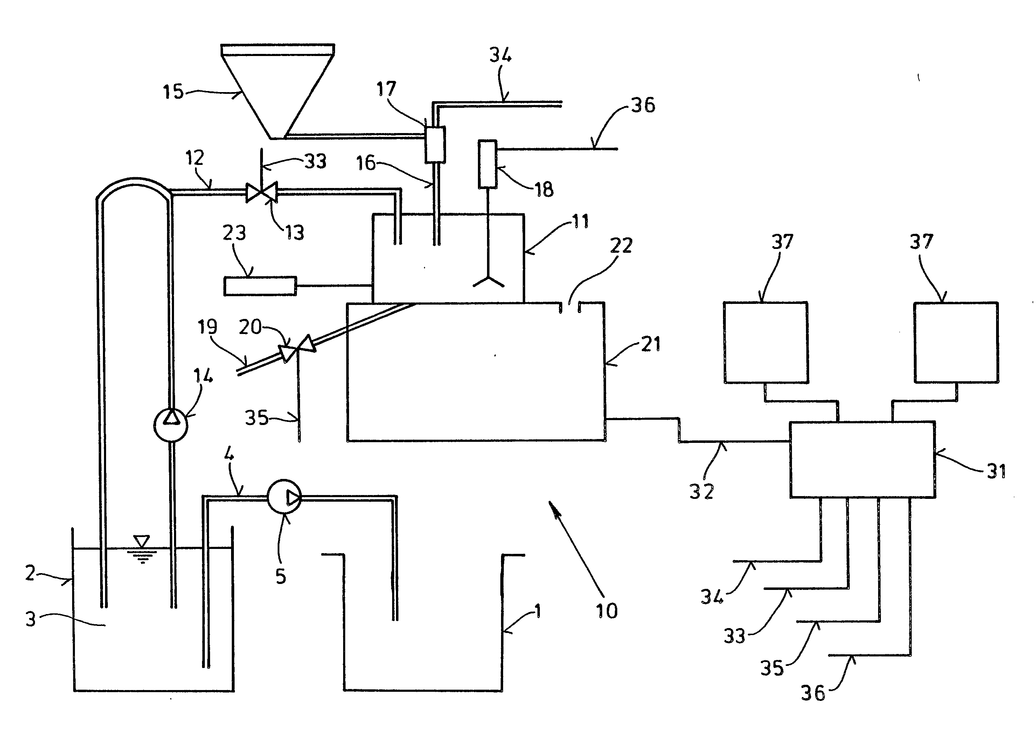

In der Zeichnung ist ein Ausführungsbeispiel einer Vorrichtung zur Bestimmung der Säuerungsaktivität in Kesselmilch dargestellt, das nachfolgend im einzelnen erläutert ist.

Die mit 10 bezeichnete Vorrichtung dient zur Bestimmung der Säuerungsaktivität der in einem Vorratstank 2 gelagerten Kesselmilch 3, die über eine mit einer Pumpe 5 versehenen Leitung 4 in einen Tank 1 eingebracht und in diesem zu Käse verarbeitet wird, und besteht im wesentlichen aus einer Küvette 11 sowie einem Farbmeßgerät 21. In die Küvette 11 kann hierbei über eine mit einem Absperrventil 13 ausgestattete Leitung 12 mittels einer Pumpe 13 eine Milchprobe aus dem Vorratstank 2 gefördert werden, außerdem ist der Küvette 11 ein Vorratsbehälter 15 für einen Redoxindikator-Farbstoff zugeordnet, der über eine Leitung 16, in die eine Dosiereinrichtung 17 eingesetzt ist, in die Küvette 11 eingebracht werden kann. Mittels eines Rührwerkes 18 können die Milchprobe sowie der Redoxindikator-Farbstoff, die gesteuert zuführbar sind, homogen miteinander vermischt werden. Über eine mit einem Ventil 20 versehene Ablaufleitung 19 ist die Küvette 11 zu entleeren.

Die Farbänderungen der Milchprobe werden mittels des Farbmeßgerätes 21 ermittelt. Dazu wird die Küvette 11 mit Hilfe einer Servoeinrichtung 23 über eine Meßöffnung 22 des Farbmeßgerätes 21 geschoben. Eine relative Verstellbarkeit zwischen dem Farbmeßgerät 21 und der Küvette 11 ist angezeigt, um dieses von Zeit zu Zeit kalibrieren zu können.

Das Farbmeßgerät 21 ist über eine Steuerleitung 32 an einen Rechner 31 angeschlossen, in dem die Farbmessungen ausgewertet werden. Und in Abhängigkeit von den Meßergebnissen kann die Kesselmilch 3, beispielsweise durch Änderung der Zugabe eines Säureweckers beeinflußt werden, um deren Säuerungsaktivitäten während der Verarbeitung in dem Tank 1 zu steuern.

Des weiteren können mittels des Rechners 31, der über Steuerleitungen 33, 34, 35 und 36 mit dem Ventil 13 der Zuführungsleitung 12, der Dosiereinrichtung 17, dem Ventil 20 der Ablaufleitung 19 sowie dem Rührwerk 18 verbunden ist, diese Aggregate ebenfalls gesteuert werden, so daß das Verfahren zur Bestimmung der Säuerungsaktivität automatisch ablaufen kann. Mittels Bildschirmen 37 ist eine Sichtkontrolle jederzeit möglich.

Die Funktionsweise der Vorrichtung 10 sowie der Arbeitsablauf des Verfahrens zur Bestimmung der Säuerungsaktivität in der Kesselmilch 3 wird nachfolgend anhand eines Beispieles im einzelnen erläutert:

Vorbereitung der Redoxindikator-Farbstofflösung:

In 200 ml, auf 60° C erwärmten destillierten Wasser werden 20 mg Resazurin (83 %) gelöst. Die Lösung muß in fest verschlossenen Flaschen bei Dunkelheit kalt gelagert werden und darf nicht älter als zwei Tage sein, da mit zunehmendem Alter die Lösung träger reagiert.

Die Lösung wird in den Vorratsbehälter 15 eingefüllt.

Probenahme:

Der Kesselmilch 3 wird unmittelbar vor dem Umpumpen aus dem Vorratstank 2 über die Zuführungsleitung 12 eine Probe von beispielsweise 20 ml entnommen und in die Küvette 11 eingebracht.

Probebereitung und Messung

In der auf 40° C angewärmten Küvette 11 werden den 20 ml der Milchprobe 2 ml der Resazurin-Lösung zugeführt und mittels des Rührwerkes 18 homogen miteinander vermischt. Danach wird mittels des Farbmeßgerätes 21 eine erste Farbmessung $(x_0)$ durchgeführt.

Die zweite und die dritte Farbmessung $(x_1$ bzw. $x_2)$ erfolgt 5 bis 10 Minuten nach der ersten Messung, wobei die Küvette 11, um einen Temperaturabfall zu vermeiden, entsprechend zu isolieren ist oder zwischen den Messungen in einem Wärmeschrank auf eine Temperatur von 37° C gebracht werden kann.

Vor der zweiten bzw. dritten Messung ist der Inhalt der Küvette 11 mittels des Rührwerkes 18 durchzumischen.

Die Milchprobe mit dem Redoxindikatorfarbstoff ist während der Reaktionszeit lichtgeschützt aufzubewahren.

Aus den Werten $x_1$ und $x_2$ der Farbmessungen errechnet sich aus der Differenz zu $x_0$ das delta $x_0$ - $x_1$ und das delta $x_0$ - $x_2$

Aus den Abweichungen kann aufgrund von Vergleichswerten auf das Säuerungsverhalten während der Käseherstellung insbesondere auf die pH- und Säuregradänderungen innerhalb von beispielsweise 120 Minuten geschlossen und es kann z. B. durch Zugabe von mehr oder weniger Säurewekkern in die Kesselmilch 3 die Säuerungsaktivität der Kesselmilch beeinflußt werden.

**Ansprüche**

1. Verfahren zur Bestimmung der Säuerungsaktivität in Kesselmilch, insbesondere in zur Weichkäseherstellung verwendbarer Kesselmilch,
**dadurch gekennzeichnet,**
daß einer der zu verarbeitenden Kesselmilch entnommenen Probe ein Redoxindikator-Farbstoff, beispielsweise Resazurin oder Methylenblau, beigegeben wird, daß die Farbe des Gemisches mittels einer ersten Farbmessung unmittelbar nach der Zugabe des Redoxindikator-Farbstoffes bestimmt wird und daß nach einer oder mehreren wählbaren Zeitspannen zur Feststellung der Stoffwechseländerungen der Milchsäurebakterien die Farbänderungen der Probe gegenüber der ersten Farbmessung und/oder vorhergehender Farbmessungen ermittelt wird.

2. Verfahren nach Anspruch 1,
**dadurch gekennzeichnet,**
daß der Kesselmilch unmittelbar vor dem Einlaben eine Probe entnommen wird.

3. Verfahren nach Anspruch 1 oder 2,
**dadurch gekennzeichnet,**
daß der Redoxindikator-Farbstoff in Form einer Lösung im Verhältnis von ca. 1:10, beispielsweise mittels einer Pipette, der Probe zugegeben, in diese homogen eingemischt und in eine vorgewärmte Küvette eingefüllt wird.

4. Verfahren nach Anspruch 3,
**dadurch gekennzeichnet,**
daß der Redoxindikator-Farbstoff im Verhältnis von ca. 1:1000 in vorzugsweise auf 60° erwärmten destillierten Wasser gelöst wird.

5. Verfahren nach einem oder mehreren der Ansprüche 1 bis 4,
**dadurch gekennzeichnet,**
daß die Farbmessungen in gleichen zeitlichen Abständen von vorzugsweise fünf Minuten vorgenommen werden.

6. Verfahren nach einem oder mehreren der Ansprüche 1 bis 5,
**dadurch gekennzeichnet,**

daß die Küvette zwischen den Farbmessungen, beispielsweise in einem Wärmeschrank, bei einer Temperatur von 30 bis 50° C gehalten wird.

7. Verfahren nach einem oder mehreren der Ansprüche 1 bis 6,
**dadurch gekennzeichnet,**
daß die Farbmessungen mitttels eines Farbmeßgerätes vorgenommen werden.

8. Vorrichtung zur Anwendung des Verfahrens nach einem der Ansprüche 1 bis 7,
gekennzeichnet durch eine mit einem Rührwerk (18) versehenen Küvette (11), der über eine Zuführungsleitung (12) aus einem Fermenter (2) oder einer Kesselmilchleitung die zu untersuchende Milch (3) vor dem Einlaben und aus einem Vorratsbehälter (15) ein Redoxindikator-Farbstoff gesteuert zuführbar sind, und einem Farbmeßgerät (21) zur Bestimmung der Farbänderungen der in der Küvette (11) hergestellten Mischung.

9. Vorrichtung nach Anspruch 8,
**dadurch gekennzeichnet,**
daß die Küvette (11) zur Vornahme von Kalibrierungen verfahrbar, vorzugsweise mittels eines Schlittens (23) auf dem Farbmeßgerät (21) verfahrbar, angeordnet ist.

10. Vorrichtung nach Anspruch 8 oder 9,
**dadurch gekennzeichnet,**
daß in der Zuführungsleitung (16) des Redoxindikator-Farbstoffes eine Dosiereinrichtung (17) angeordnet ist.

11. Vorrichtung nach einem oder mehreren der Ansprüche 8 bis 10,
**dadurch gekennzeichnet,**
daß das Farbmeßgerät (21) an einen Rechner (31) zur Auswertung der Farbmessungen angeschlosen ist.

12. Vorrichtung nach Anspruch 11,
**dadurch gekennzeichnet,**
daß mittels des Rechners (31) die Zugabemenge der zu untersuchenden Milch (3) und des Redoxindikator-Farbstoffes in die Küvette (11) und/oder eines Säureweckers in die zu verarbeitende Milch und/oder deren Reifezeit steuerbar sind.